# FASCICULE DE BREVET EUROPEEN

(11) **EP 1 682 156 B1**
(45) Date de publication et mention de la délivrance du brevet: **24.01.2007**
(21) Numéro de dépôt: 04805452.2
(22) Date de dépôt: 15.11.2004
(51) Int. Cl.: A61K 31/575, C07J 51/00

(54) **COMPLEXES A BASE DE STRONTIUM, COMPOSITIONS PHARMACEUTIQUES ET PRODUITS DIETETIQUES**
KOMPLEXE AUF STRONTIUMBASIS, PHARMAZEUTISCHE ZUSAMMENSETZUNGEN UND DIETÄTISCHE PRODUKTE
STRONTIUM-BASED COMPLEXES, PHARMACEUTICAL COMPOSITIONS AND DIETETIC PRODUCTS

(30) Priorité: 14.11.2003 FR 0313357
(43) Date de publication de la demande: 26.07.2006
(73) Titulaire: Medesis Pharma S.A., 75002 Paris (FR)
(72) Inventeur: MAUREL, Jean-Claude, F-34160 Castries (FR); CUDENNEC, Claude-Alain, F-76230 Bois-Guillaume (FR); POUCHERET, Patrick, Résidence Cévennes-Bâtiment L1, F-34000 Montpellier (FR)
(74) Mandataire: Tezier Herman, Béatrice
(86) Numéro de dépôt international: PCT/FR2004/002912
(87) Numéro de publication internationale: WO 2005/049038

(56) Documents cités:
- WO-A-96/23811
- WO-A-98/01461
- FR-A- 2 658 186
- US-A- 5 075 336
- REGINSTER J-Y: "Strontium ranelate in osteoporosis" CURRENT PHARMACEUTICAL DESIGN, BENTHAM SCIENCE PUBLISHERS, SCHIPHOL, NL, vol. 8, no. 21, 2002, pages 1907-1916, XP008024667 ISSN: 1381-6128 cité dans la demande

## Description

La présente invention concerne des complexes organiques à base de sitostérols, d'acylglycérols et de strontium et leur utilisation dans le domaine pharmaceutique et l'industrie diététique. Elle a trait également à des méthodes pour traiter diverses maladies, en particulier des maladies osseuses et sanguines, par administration de ces complexes. Elle concerne aussi des compositions pharmaceutiques contenant ces complexes, en particulier pour traiter les maladies osseuses, telles que l'ostéoporose, et pour traiter des hémopathies.

Les acylglycérols, plus particulièrement les acylglycérols d'acides gras, sont présents dans la plupart des végétaux et sont des constituants majoritaires des corps gras végétaux et animaux. Seuls varient d'un acylglycérol à l'autre le nombre d'acides gras, leur position sur le glycérol, leur longueur de chaîne et le nombre de leurs éventuelles insaturations. Il peut s'agir, en particulier, de mono-, di- ou triacylglycérols. Le sitostérol, dont il existe deux isomères actifs le γ et le β sitostérol, est, lui aussi, un constituant de l'ensemble des végétaux. Certains extraits totaux de plantes qui, comme la majorité des plantes, contiennent parmi leurs très nombreux constituants des flavonoïdes, tannins, saponines, coumarines, alcaloïdes, triterpènes, stérols, carbohydrates et/ou glycosides ont été décrits pour leur activité thérapeutique, en particulier hypoglycémiante, comme par exemple l'extrait d'accacia (Egypt. J. Pharm. Sci., 1992, 33 (1-2), 327-340), ou l'extrait de teucrium oliverianum (Fitoterapia, 1984, 55(4), 227-230). Aucun lien entre l'activité et la fraction contenant les stérols n'est établi. Les travaux des auteurs de la présente demande les ont déjà conduit à décrire, dans différents brevets, différentes associations, en particulier sous forme de complexes organométalliques utilisables dans le traitement et/ou la prévention de l'hyperglycémie, du diabète et des maladies connexes. On citera en particulier la demande de brevet WO 96/23811 qui décrit des complexes organométalliques à base de sitostérols et d'acylglycérols, le métal étant en particulier le vanadium.

L'ostéoporose est une maladie caractérisée par une masse osseuse faible et une détérioration du tissu osseux, conduisant à une fragilité osseuse et ainsi à une augmentation du risque de fractures des os. L'ostéoporose peut toucher l'ensemble du squelette et cause des fractures essentiellement au niveau de la hanche, du poignet ou du rachis dorsal ou lombaire. L'ostéoporose est un problème de santé publique majeur et global, accru par le vieillissement de la population. En outre, même si l'ostéoporose est souvent reliée aux personnes de plus de 50 ans, cette maladie peut arriver à tout âge. Certains facteurs sont liés au développement de l'ostéoporose ou contribuent à la probabilité de développer cette maladie, on les appelle les facteurs de risque. Ainsi, il est établi qu'une femme est plus susceptible d'être atteinte de cette maladie qu'un homme, il en est de même pour les personnes âgées, les os devenant moins denses et donc plus fragiles avec l'âge, pour les personnes fines et/ou de petites tailles, etc. Chez l'adulte, le capital osseux est la résultante de l'équilibre entre la résorption osseuse par les ostéoclastes et la formation osseuse par les ostéoblastes jusqu'à l'âge de 30 ans, la densité osseuse commençant à diminuer à partir de cet âge. A la ménopause, la perte osseuse chez la femme est accélérée avec la déficience en oestrogène qui augmente l'activité des ostéoclastes, conduisant alors à une résorption osseuse plus importante que la formation osseuse. L'aménorrhée, les taux bas en oestrogène, le taux bas en testostérone chez l'homme, l'anorexie, certains traitements médicamenteux (tels que les glucocorticoïdes, certains anti-convulsifs), un régime alimentaire à faibles doses en calcium et vitamine D, le tabagisme ou l'usage excessif d'alcool sont des facteurs de risque supplémentaires.

Un certain nombre de traitements thérapeutiques préventifs ou curatifs contre l'ostéoporose sont décrits ou actuellement utilisés, tels que des biphosphonates, la calcitonine, des traitements hormonaux (oestrogène et progestin), des modulateurs sélectifs au récepteur à l'oestrogène (raloxifène).
Un nouveau médicament en cours d'enregistrement dans le traitement de l'osteoporose utilise le strontium comme principe actif. Ainsi, certaines publications, notamment Reginster J.-Y., Current Pharmaceutical Design, 2002, 8, 1907-1916, décrivent l'activité du ranelate de strontium dans le traitement de l'ostéoporose. Une étude de deux ans en double aveugle sur des femmes post-ménopausées ayant pris une dose de 2g/jour de ranélate de strontium a montré une augmentation significative de la densité minérale osseuse au niveau des vertèbres lombaires. En outre, il a été observé, durant cette étude, que la phosphatase alkaline osseuse (marqueur de la formation osseuse) a augmenté de manière significative et qu'un marqueur de la résorption osseuse a diminué, ceci montre que le ranélate de strontium joue sur les deux activités biologiques, ce qui aboutit à une augmentation de la densité osseuse.
Des travaux réalisés avec le chlorure de strontium (strontium chloride) et avec le ranélate de strontium ont démontré une double action sur les effecteurs cellulaires de l'équilibre osseux: stimulation de la multiplication des ostéoblastes, et inhibition de la multiplication des ostéoclastes.
L'activité thérapeutique chez l'animal nécessite des doses élevées de strontium (200 mg de ranélate de strontium/jour, correspondant à 68,2 mg de strontium métal) et s'accompagne d'une fixation de strontium dans l'os relativement importante, avec un tum-over d'élimination qui induit un effet toxique (lyse ostéomalacique de l'os) à partir de 800 mg/jour de ranélate (4 fois la dose active). Cet effet délétère de fixation de strontium dans l'os n'est pas considéré en deçà de ces doses comme toxique car il n'altère pas les qualités mécaniques de l'os.
Toutefois, il apparaît, selon certains auteurs, que le strontium peut avoir des effets néfastes lorsque la quantité fixée dans l'os devient trop élevée, en particulier lors d'une altération de la fonction rénale (Martine Cohen-Solal, Nephrol Dial Transplant (2002) 17 [Suppl. 2] : 30-34).

Des travaux récents ont démontré qu'il existait une corrélation entre le nombre d'ostéoblastes et le nombre de cellules souches hématopoïétiques. Les procédés induisant une augmentation du nombre des ostéoblastes induit une augmentation simultanée des cellules souches hématopoïétiques ("Osteoblastic cells regulate the haematopoietic stem cell niche", Calvi L.M. et al., *Nature. 2003 Oct 23;425(6960):841-6.* et "Identification of the haematopoietic stem cell niche and control of the niche size", Zhang J. et al., Nature. 2003 Oct 23;425(6960):836-41).
La stimulation de la reproduction des ostéoblates par le strontium est donc susceptible d'induire une stimulation des cellules souches hématopoïétiques.

Disposer d'un actif pharmaceutique utilisable à plus faibles doses, tout en présentant une activité pharmacologique au moins équivalente à celle des sels de strontium utilisés à ce jour, serait d'un grand intérêt thérapeutique (diminution des risques néfastes) et économique.

La présente invention propose donc de nouveaux complexes organiques à base de sitostérols, d'acylglycérols et de strontium permettant d'augmenter la biodisponibilité du strontium. Les auteurs de la présente invention ont en effet constaté qu'en administrant à un animal des complexes organiques particuliers contenant du strontium, une croissance osseuse est constatée, même à de faibles doses en strontium, tout en présentant une moindre fixation du strontium à l'os.

La présente invention a donc pour objet, selon un premier aspect, de nouveaux complexes organométalliques susceptibles d'être obtenus par réaction de:
- Au moins un cation de strontium,
- Le sitostérol ou un extrait végétal en contenant,
- Au moins un mono-, d'un di- ou d'un triglycéride répondant à la formule (I): 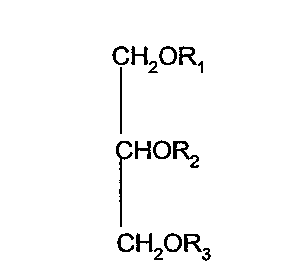
dans laquelle:
- R1 est un reste acyle d'acide gras en C14 à C24 saturé ou non, linéaire ou ramifié, un atome d'hydrogène, ou un mono-, un di- ou un tri- galactose ou glucose,
- R2 est un reste acyle d'acide gras en C2 à C18, linéaire ou ramifié, saturé ou non,
- R3 est un reste acyle d'acide gras en C14-C24 saturé ou non, linéaire ou ramifié, ou un atome d'hydrogène.

Selon une variante préférée, l'un au moins des groupements R1 ou R3 de la formule (I) ci-dessus est constitué d'un reste acyle d'acide oléique (C18: 1[cis]-9).
Selon une autre variante préférée, R2 présente une insaturation, de préférence il est un reste acyl d'acide gras en C18, avantageusement il représente un reste d'acide oléique ou d'un de ses isomères de position de la double liaison (cis-6, 7, 9, 11, 12 et 13) ou d'un de ses isomères isoramifiés.
Selon une autre variante, R2 représente un groupe acétyle.
Selon une variante particulièrement avantageuse de l'invention, R3 de la formule (I) ci-dessus représente l'atome d'hydrogène.

Les complexes de l'invention se forment relativement aisément par mélange des trois types de constituants en les maintenant généralement à une température comprise entre 30°C et 40°C pendant une douzaine d'heures ; la réaction catalytique de fabrication du complexe se fait de façon plus lente à température ambiante (5 à 8 jours à 25°C) ; le complexe ne se fabrique que peu ou pas si le mélange est maintenu au froid.

Ainsi donc, la demanderesse a clairement mis en évidence l'effet favorable d'une élévation de température, en particulier entre 30 et 40°C, sur la réaction intervenant entre les constituants du complexe de l'invention.

Les trois types de composés réagissent très aisément, par leur mise en solution dans un solvant organique, par exemple le dichlorométhane, l'éther, le chloroforme, le méthanol, l'éthanol ou l'acétate d'éthyle qui seront évaporés par la suite.

Le cation du strontium utilisable pour préparer les complexes selon l'invention est donc un cation divalent susceptible de se complexer avec les deux types de dérivés organiques ci-dessus. A titre d'exemples de sels de strontium susceptibles d'être utilisés dans la présente invention pour fournir le cation de strontium, on peut notamment citer les dihalogénures, plus spécifiquement le dichlorure, les sulfates, les hydrates, qui peuvent être avantageusement dissous dans l'eau ou parfois dans des alcools. On peut encore citer des dérivés organiques de strontium comme les acétylacétonates, les alcoolates, en particulier le ranélate de strontium, ou des complexes de strontium avec des solvants organiques, par exemple des éthers, le THF, la DMF. Ces dérivés organiques de strontium sont généralement solubles dans des solvants organiques, plus particulièrement dans des solvants chlorés comme le chloroforme ou le dichlorométhane.

Le sitostérol utilisé pour la préparation des complexes de l'invention peut être sous forme β ou γ. On peut également utiliser un mélange de ces deux stérols.

Le sitostérol existe sous forme commerciale. Mais il est alors généralement en mélange avec du carnpestérol. Dans ces produits commerciaux généralement extraits du soja, le β-sitostérol ne représente que 50% du produit, l'impureté principale étant le campestérol. Une autre origine commerciale intéressante est obtenue à partir du tall-oil, dans laquelle le sitostérol représente plus de 75 % du produit. On pourra obtenir du β-sitostérol présentant une pureté supérieure à 95 %, voir même de 99 %, en procédant de la façon suivante : on fait plusieurs recristallisations successives à l'acétone du mélange commercial, ce qui permet de prépurifier le β-sitostérol par élimination du campestanol et du sitostanol présents dans le mélange. Puis, on fait subir au produit ainsi prépurifié 1 à 3 étapes de purification par chromatographie liquide haute pression sur colonne préparative C18 en utilisant des mélanges éluants, tels que le méthanol, en particulier du méthanol à 100 % ou des mélanges de méthanol et d'acétonitrile, en particulier des mélanges 80-20 ou tout mélange intermédiaire permettant d'obtenir le sitostérol avec une pureté supérieure à 95 voir même à 99 %. Cette pureté est déterminée par chromatographie en phase gazeuse.

On peut également préparer le sitostérol par extraction à partir de végétaux selon des techniques décrites dans la littérature, par exemple P95 de la thèse présentée à Montpellier par Claude Cerdon ayant pour titre : « Modulation de la production de sapogénines stéroïdiques en réponse à l'inhibition de la synthèse de stérols ».

Cette extraction est avantageusement réalisée par complexation des métaux selon le procédé décrit en particulier dans le brevet français N°. 2 316 247 dans lequel est décrit un procédé pour isoler les 3-hydroxystéroides et les 3 oxostérodes de mélange contenant ces composés.

Pour réaliser cette extraction, on pourra utiliser tout végétal ou produit d'origine végétale connu pour sa teneur relativement élevée en sitostérol.

A titre d'exemples de végétaux ou de produits d'origine végétale à teneur relativement élevée en sitostérol libre, on citera en particulier l'huile d'olive, l'huile de soja, les feuilles de coton, les feuilles de café, les germes de blé.

Il faut préciser cependant que la fraction sitostérol libre contient une proportion variable selon les végétaux des isomères 24 R et 24 S, proportion mal connue car peu ou pas étudiée, et qui paraît expliquer une meilleure activité relative de la fraction stérol de certains végétaux et surtout l'excès de sitostérol nécessaire lors de la fabrication des nouveaux produits.

Les acylglycérols de formule (I) utiles pour la préparation des complexes selon l'invention peuvent être isolés dans la plupart des végétaux.

Plus généralement, la fraction chimique contenant ces produits peut être extraite de labiées, ortie (urtica dioica et urens), de la sauge, les bugles, la luzerne ou alfalfa (medicago sativa), les eucalyptus (globulus, delegatensis), l'angelica archangelica et l'angelica sinensis, les ombelliferes, le gymnema sylvestre (asclepiadaceae), le marsdenia condurango, le momordica charantia, le gingko biloba, le chardon-marie, le thé vert, le thé noir (camelia sinensis), la rhubarbe, le dioscorea dumetorum (dioscoreaceae), l'indigofera arrecta (papilionaceae), le pittosporaceae, l'agrimonia eupatoria, le curcuma xanthorrhiza (roxb.), l'uncaria gambier (roxb.), le swertia chirayita (roxb.), les résédacées (réséda, phyteuma, lutea, alba, luteola), l'harpagophytum, les rubiacées, les gentianxcaes, l'asparagus racemosus, le dioscorea dumetorum (dioscoracese), l'aubépine (crataegus oxyacantha), le gui (viscum album), les palétuviers (rhizophoracées), les palmiers, le kaki, les chênes, le chêne à galle (fagacées), les ronces, l'hamamélis (hamamélidacées), le ratanhia (kramériacées), le salicaire (lythracées), le calophyllum (clusiacées), les acacias, l'acacia à cachou (Légumineuses mimosoidées), le quebracho (anacardiacées), les raisins (vitis vinifera, ampelidacées), les cassis (saxifragacées dont ribes nigrum), les myrtilles (erieaeées), les mûres (rubus fructicosus), le sureau, le choux-rouge, l'ail (allium sativum), le coriendre (coriandrum sativum), le juniper (juniperus communis), les pins (abiétacées), le pin maritime, les cyprès (cupressasées), les hibiscus, les rhus (anacardiacae), les dicotylédones, les fougères, les gymnospermes, les melianthus, les rosasées, les roses, l'eriobotrya japonica (rosaceae), le boussingaultia baselloides, le malva verticillata (malvaceae), les fraisiers, les citrus (rutaeées), la benoite, le blighia sapida (sapindaceae), l'aubépine, les châtaigniers (fragacées), les sumacs (anacardiacées), les myrabolans (combrétacées), la bistorte, les légumineuses césalpinioidées (dividivi, tara, algarobille), les légumineuses papilioncées (derris, lonchcarpus, mundelea, tephrosia), le lespedeza, le sophora, les polygonacées, les légumineuses, le sarrasin.

On utilise de façon particulièrement avantageuse comme source d'acylglycérols des huiles végétales insaturées, en particulier de l'huile d'olive de première pression à froid.

D'une façon générale, on choisira comme source d'acylglycérols utiles selon l'invention une huile ou une fraction d'huile contenant une forte teneur en acide oléique, une telle huile contenant généralement une forte proportion d'acylglycérols utiles selon l'invention.

A titre d'exemples de telles huiles, on citera:
- l'huile d'olive dont la teneur en acide oléique (C18: 1) est comprise entre 60 et 80 %, les huiles européennes étant plus riches en Clg: 1 que les huiles d'Afrique du Nord,
- l'huile de tournesol de variété dite hybride tournesol oléique, contenant 83 % de C18:1 au lieu de 16 % dans l'huile de tournesol normale,
- l'huile de carthame de variété oléique, contenant de 73 à 80 % de C 18:1 au lieu de 10 à 20 % dans la variété linoléique,
- l'huile d'amande contenant de 64 à 82 % de C18:1,
- l'huile de noisette contenant de 66 à 83 % de C18:1,
- l'huile d'avocat dont la teneur en C18:1 varie entre 36 et 80 %.

La fraction contenant des acylglycérols utiles pour préparer les complexes selon l'invention peut être avantageusement préparée à partir de l'huile d'olive de la façon suivante : on effectue une prépurification de l'huile d'olive en la passant sur une faible hauteur de silice (10 à 15 cm), en réalisant le vide et en éluant à l'aide d'un solvant organique tel que le dichlorométhane ou un mélange de cyclohexane et d'acétate d'éthyle dans des proportions 96/4 ou tout autre éluant présentant une polarité voisine, de façon à isoler les triglycérides présents dans l'huile parmi lesquels on trouve celui présentant un C18 sur le C en position 2 du glycérol. La silice est ensuite lavée avec de l'acétate d'éthyle afin de récupérer les monoglycérides et les diglycérides intéressants selon l'invention, c'est-à-dire ceux comportant un acide gras en C18 sur le carbone en position 2 dans le groupement glycérol.

La fraction ainsi obtenue est ensuite passée sur une colonne de silice et éluée avec différents gradients de mélange acétate d'éthyle/cyclohexane compris entre 10/90 et 100/0 de façon à séparer les différentes familles chimiques de l'huile et à récupérer la famille active recherchée.

Selon un aspect particulier de l'invention, les acylglycérols utilisés dans la cadre de la présente invention sont choisis parmi le 1,2-dioléine et 1-oléoyl-2-acétyl glycérol.

Les acylglycérols de l'invention sont également disponibles commercialement. En particulier, le 1-oléoyl-2-acétylglycérol et le 1,2-dioléoylglycérol sont disponibles commercialement à un taux de pureté élevé (plus spécifiquement, le monooléate de glycérol contenant environ 44 % de glycérides dioléiques, parmi lesquels environ 14 % est le 1,2-dioléine). Un tel composé est accepté pharmaceutiquement *(European Pharmacopeia* (4^{th} Edition), *USP* 25/NF20, et *Japanese Standard of food Additives)* et est notamment commercialisé par la société Gattefossé sous le nom PECEOL®.

Les complexes selon l'invention sont, comme on l'a vu précédemment, aisément fabriqués par simple mélange des trois types de composés décrits ci-dessus. Ce mélange est avantageusement réalisé dans un solvant organique tel que le dichlorométhane, l'éther, le chloroforme, l'acétate d'éthyle, l'éthanol, le mélange est ensuite maintenu à une température comprise entre 30°C et 40°C pendant une douzaine d'heures, et une durée inférieure lorsque l'on associe une agitation du mélange.

On utilise avantageusement des proportions équimolaires des deux constituants lipidiques ; toutefois, ces conditions ne sont pas critiques, et l'on pourra avantageusement utiliser un excès de sitostérol par rapport à l'acylglycérol dans des proportions de 1 à 50 en fonction de la nature du stérol (24 R ou 24 S).

Le métal (strontium) peut être utilisé à doses très basses par rapport aux deux autres constituants, en particulier dans un rapport de 1/10 à 1/100 exprimé en moles par rapport à l'acylglycérol.

Les différents constituants sont formellement identifiés par des moyens analytiques adaptés:
- pour le sitostérol: chromatographie en phase gazeuse,
- pour l'acylglycérol HPLC avec un détecteur à diffusion de lumière, sur une colonne de kromasil C18, en présence d'un éluant constitué par exemple d'acétonitrile isocratique.
On peut également utiliser la chromatographie en phase gazeuse pour l'identification des monoglycérides.

Le pic de masse du complexe n'est généralement pas détectable avec les méthodes usuelles, telles que l'ionisation chimique et l'impact électronique, ce qui peut s'expliquer par le fait que les complexes formés par ces deux constituants avec le métal sont généralement instables comme la plupart des complexes organométalliques ayant une activité biologique.

Selon un autre de ses aspects, l'invention concerne l'utilisation des complexes décrits précédemment en tant qu'agents transporteurs de cations du strontium, les ligands liés au strontium servant à accroître la biodisponibilité dudit strontium.

Une telle application est d'autant plus importante que, d'une façon générale, l'homme du métier sait que la difficulté d'utilisation thérapeutique du strontium peut être liée à sa toxicité au delà des doses actives.

Les complexes organométalliques de strontium, décrits selon l'invention, permettent d'obtenir une activité de croissance osseuse au moins équivalente à celle du dichlorure de strontium à des doses de strontium (exprimée en métal) 10, voire 100 à 500 fois plus faibles que celle utilisée dans le cas du dichlorure de strontium. De plus l'activité pharmacologique est obtenue avec une moindre fixation du strontium dans l'os. On peut alors affirmer qu'à ces doses, le strontium sous forme de complexe est dénué de toxicité.

Les complexes organométalliques décrits dans la présente invention optimisent ainsi la biodisponibilité du strontium transporté, permettant son utilisation thérapeutique avec une efficacité renforcée une toxicité faible ou nulle, ce qui présente un avantage considérable par rapport à l'état de la technique.

Selon un autre de ses aspects, l'invention concerne également des compositions pharmaceutiques contenant au moins un complexe tel que défini précédemment et un véhicule, excipient ou support pharmaceutiquement acceptable.

Comme excipient, véhicule ou support pharmaceutiquement acceptable, on peut utiliser tout excipient, véhicule ou support bien connu de l'homme de l'art. On peut citer, par exemple, et de façon non limitative: le lactose, l'amidon de mais, le glucose, la gomme arabique, l'acide stéarique ou le stéarate de magnésium, la dextrine, le mannitol, le talc, les huiles d'origine naturelle riches en acides gras insaturés essentiels et en stérol. En particulier, si cela s'avère éventuellement nécessaire, on peut utiliser d'autres additifs bien connus de l'homme de l'art tels que des stabilisants, des desséchants, des liants, des tampons de pH.

Les compositions de l'invention peuvent être administrées de différentes manières, en particulier par voie intramusculaire, sous-cutanée, sublinguale, per os, permuqueuse, transdermique (en administrant la composition sous forme de patch ou de gel).

Selon un autre de ses aspects, l'invention concerne l'utilisation des complexes de l'invention pour la préparation d'un médicament destiné à être utilisé comme agent régulateur ou stimulant de la croissance osseuse, en particulier dans le traitement ou la prévention des déficits ou des dysfonctionnements de la croissance osseuse, notamment dans le traitement ou la prévention de l'ostéoporose.
Selon un autre de ses aspects, l'invention concerne l'utilisation des complexes de l'invention pour la préparation d'un médicament destiné à être utilisé pour stimuler la fabrication de cellules souches hématopoïétiques, en particulier dans le traitement des pathologies sanguines s'accompagnant d'un déficit hématopoïétique, plus spécifiquement en complément des chimiothérapies anticancéreuses.

L'invention concerne également une méthode de traitement des pathologies ou désordres cités ci-dessus, comprenant l'administration à un sujet, notamment humain, d'une dose efficace d'un complexe ou d'une composition pharmaceutique tels que définis ci-avant.

Les dosages et les régimes dans lesquels le complexe est administré varient selon la formulation, le mode d'administration, les conditions et les particularités du sujet à traiter. A titre de comparaison, le ranélate de strontium peut être administré à un animal dans des quantités qui varient entre environ 200 et 1 800 mg/Kg/jour. Chez l'homme, la quantité de ranélate de strontium nécessaire pour obtenir une activité thérapeutique est de 2 g/jour. Lorsque le strontium est lié au complexe décrit dans l'invention nécessaire pour obtenir une activité thérapeutique sont divisées par 10 à 100, du fait d'une meilleure biodisponibilité du métal.

A cet égard, les termes "traitement" ou "traiter" incluent des traitements aussi bien curatifs que prophylactiques. Le complexe selon l'invention peut être utilisé à un stade précoce de la maladie, ou avant l'apparition des premiers symptômes, ou encore à un stade avancé de la maladie.

Dans toutes ces utilisations, on met en application le fait que la biodisponibilité du strontium lui-même connu comme stimulant de la croissance osseuse se trouve accrue du fait de sa complexation.

La complexation conduit dans tous les cas à une exaltation du pouvoir biocatalytique du strontium, ce qui permet d'obtenir une activité avec des doses de strontium considérablement diminuées par rapport aux doses habituellement utilisées.

Selon un autre de ses aspects particulièrement important, l'invention concerne également des produits diététiques, notamment des produits diététiques utilisables comme compléments alimentaires à activité régulatrice du métabolisme du calcium et/ou protectrice de la dégradation osseuse, en particulier de la densité et/ou de la qualité osseuse, incorporant les complexes précédemment définis ainsi qu'un mode de préparation de ces produits.

En effet, étant donné le mode de préparation des complexes décrits précédemment, ils peuvent se former aisément par addition, dans une huile riche en acides oléiques (C18: 1), de sitostérol ou un extrait végétal contenant au moins l'une de ces deux formes de sitostérol et d'un sel de métal tel que défini précédemment.

Comme on l'a vu précédemment, les teneurs en acide oléique C18: 1 des huiles végétales varient considérablement selon la nature du végétal et son origine géographique.

Pour la préparation des produits diététiques selon l'invention, on utilise avantageusement une huile végétale dans laquelle les acides oléiques représentent au moins 60 % des acides gras.

Selon une variante préférée, on utilisera de l'huile d'olive, de préférence de l'huile de première pression à froid.

Le sitostérol sera avantageusement introduit sous forme d'un extrait végétal. On pourra, en particulier, utiliser un extrait végétal du commerce obtenu à partir du soja ou du tall-oil.

Le produit diététique est obtenu par simple mélange de l'huile, de l'extrait végétal riche en sitostérol et du cation de strontium, puis chauffage et éventuellement agitation du mélange.

A titre d'exemple de tels produits diététiques selon l'invention, on citera les mélanges constitués de:
- 100 ml d'huile dont les acides gras ont une teneur d'au moins 60 % en acides oléiques, en particulier de l'huile d'olive,
- 20 g de sitostérol extrait du tall-oil,
- 2 à 8 g de sel de strontium exprimé en poids de métal

Ce produit administré quotidiennement à raison de 5 ml per os permet une meilleure régularisation du métabolisme du calcium et une prévention de la dégradation osseuse (densité et/ou qualité). Un tel traitement diététique est particulièrement indiqué dans le cas des femmes post-ménopausées en prévention des fractures vertébrales et osseuses.

Les exemples suivants sont donnés à titre purement illustratif de l'invention.

### EXEMPLES

### Exemple 1 : Préparation du complexe selon l'invention

1,70 g de sitostérol commercial dissout dans 10 ml d'éthanol.
100 mg d'un mélange commercial de 1,2-dioleine et 1,3 dioleine.
840 mg de strontium sous forme de sulfate solubilisé dans 5 ml d'eau purifiée.
70 ml d'huile de soja.
Le mélange est agité et chauffé pendant 30 minutes. L'éthanol est ensuite évaporé.

### Exemple 2: Préparation du complexe selon l'invention

1,70 g de sitostérol commercial dissout dans 10 ml d'éthanol.
840 mg de strontium sous forme de sulfate solubilisé dans 5 ml d'eau purifiée.
2 ml d'un extrait purifié de mono et diglycérides extraits de l'huile de palme-palmiste (70 à 80 % d'acide oléique).
Le mélange est agité et chauffé pendant 30 minutes à 35°C. L'éthanol est ensuite évaporé.

### Exemple 3: Préparation d'une composition pharmaceutique selon l'invention

Une composition pharmaceutique est préparée à partir du complexe formé selon l'exemple 2. Le produit est introduit dans des capsules gastro-résistantes. Le produit est ensuite administré.

### Exemple 4: Préparation d'une composition pharmaceutique selon l'invention

Une composition pharmaceutique est préparée à partir du complexe formé selon l'exemple 1. Le produit est liquide et se présente sous la forme d'une émulsion. Il est prêt à être administré *per os* chez l'homme, ou via la voie IP ou la voie rectale chez l'animal.

### Exemple 5: Préparation d'une composition pharmaceutique oro-dispersible selon l'invention

Une composition pharmaceutique est préparée à partir du complexe formé selon l'exemple 2 en rajoutant au mélange des excipients adaptés à cette forme pharmaceutique.

### Exemple 6: Tests Pharmacologiques

Le complexe selon l'exemple 4 est évalué selon le protocole suivant:
Administration chez le rat Wistar femelle de poids 200 g.

| | | |
|---|---|---|
| Nombre d'animaux: 30. | | |
| Nombre d'animaux par lot: 6 et 8. | | |
| Identification des Lots: | Lot #1 = Contrôle (émulsion huileuse) | C (n=6), |
| | Lot #2 = Contrôle Chlorure de Strontium | SrCl (n=8) |
| | Lot #3 = Traité NP05 faible dose | NP05-f (n=8) |
| | Lot #4 = Traité NP05 dose élevée | NP05-F (n=8) |

| **Traitement** | | |
|---|---|---|
| Complexe Testé: | NP05 (composition fabriquée selon l'invention, dans laquelle varie uniquement la quantité de strontium): | |
| Principe Actif: | Strontium. | |
| Préparation: | selon l'exemple 4 | |

| Dosage principe actif: | **métal Strontium: (mg/kg/j)** | |
|---|---|---|
| | C | **0** |
| | SrCl | **68,2** |
| | NP05-F | **68,2** |
| | NP05-f | **12,15** |
| Volume d'administration: | 2 ml/kg. | |
| Voie d'administration: | Voie rectale. | |
| Fréquence d'administration: | Une fois par jour - toute les 24 heures. | |
| Durée du traitement : | 21 jours. | |

Echantillons de tissus
- L'os du fémur est prélevé, nettoyé de ses adhérences tendineuses et musculaires, puis congelé après euthanasie des animaux.
- Les os sont ensuite soumis à un dosage du strontium par spectrométrie d'absorption atomique.
- Le diamètre de l'épiphyse du fémur est mesuré.
- Le dosage du strontium dans les os est réalisé.

**Les résultats** sont rassemblés dans les tableaux 1 et 2 suivants.

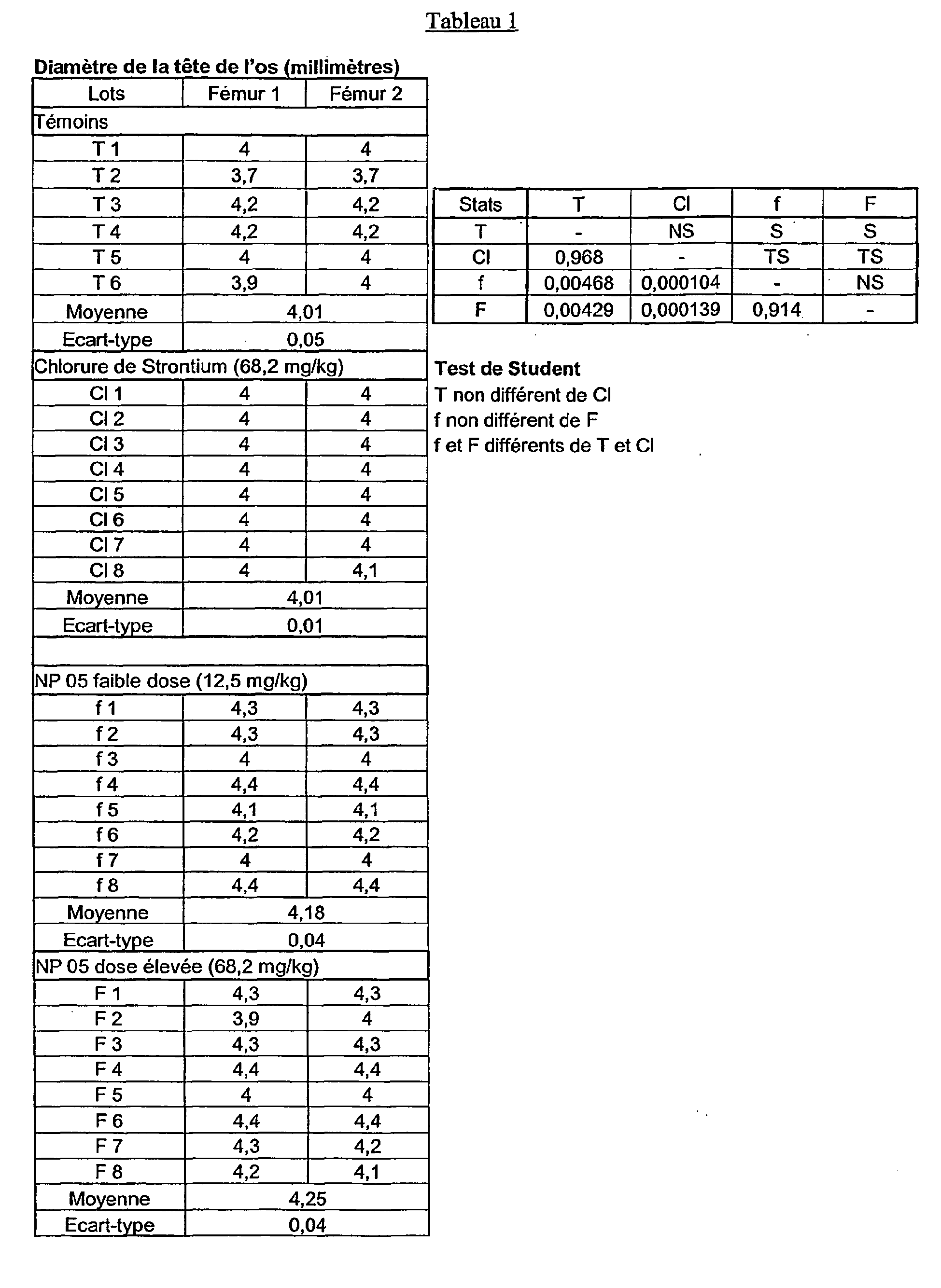

**Tableau 2**

| **DOSAGE CALCIUM** | | **DOSAGE STRONTIUM** | |
|---|---|---|---|
| Echantillon | Concentration en Ca (mg/g) | Echantillon | Concentration en Sr (mg/g) |
| T1 | 72,698 | T1 | 100,14 |
| T2 | 61,893 | T2 | 72,83 |
| T3 | 78,219 | T3 | 86,60 |
| T4 | 68,615 | T4 | 72,49 |
| T5 | 63,916 | T5 | 67,84 |
| T6 | 61,893 | T6 | 70,57 |
| Moyenne | **67,873** | Moyenne | **78,41** |
| Ecart-type | **6,604** | Ecart-type | **12,48** |
| | | | |
| C11 | 77,882 | C11 | 308,32 |
| C12 | 79,211 | C12 | 350,27 |
| C13 | 72,946 | C13 | 306,08 |
| C14 | 57,229 | C14 | 342,23 |
| C15 | 64,538 | C15 | 250,72 |
| C16 | 71,630 | C16 | 241,67 |
| C17 | 70,192 | C17 | 437,51 |
| C18 | 71,221 | C18 | 441,82 |
| Moyenne | **70,606** | Moyenne | **334,83** |
| Ecart-type | **7,069** | Ecart-type | **75,18** |
| | | | |
| f1 | 84,180 | f1 | 176,64 |
| f2 | 78,618 | f2 | 120,78 |
| f3 | 66,324 | f3 | 126,75 |
| f4 | 62,572 | f4 | 102,72 |
| f5 | 64,209 | f5 | 99,93 |
| f6 | 73,896 | f6 | 87,04 |
| f7 | 60,142 | f7 | 109,39 |
| f8 | 61,336 | f8 | 119,51 |
| Moyenne | **68,910** | Moyenne | **117,84** |
| Ecart-type | **8,908** | Ecart-type | **27,04** |
| | | | |
| F1 | 69,516 | F1 | 148,20 |
| F2 | 70,165 | F2 | 194,70 |
| F3 | 72,865 | F3 | 123,21 |
| F4 | 53,810 | F4 | 85,91 |
| F5 | 73,689 | F5 | 190,64 |
| F6 | 56,494 | F6 | 166,95 |
| F7 | 72,791 | F7 | 150,51 |
| F8 | 59,073 | F8 | 141,94 |
| Moyenne | **66,050** | Moyenne | **150,26** |
| Ecart-type | **8,185** | Ecart-type | **35,52** |

### Commentaires et résultats :

Dans cette étude, la durée de l'administration a été de 3 semaines, et les animaux traités par le dichlorure de strontium n'ont eu aucune action sur leur croissance osseuse, en même temps que la fixation du strontium dans l'os était relativement importante par rapport aux animaux témoins (x 4) ; alors que les études chez l'animal (Optimizing bone metabolism in osteoporosis : insight into the pharmacologic profile of strontium ranelate ; Marie PJ ; *Osteoporos Int 2003 Mar ;14 Suppl 3 : 9-12)* ont démontré sur le modèle des rats en croissance une activité de stimulation de la croissance osseuse lorsque l'on administre aux animaux une dose minimum de 68,2 mg de strontium (métal) sous forme de chlorure ou de ranélate pendant une durée de 8 semaines.
Par contre, les animaux traités pendant seulement 3 semaines par le strontium préparé selon l'invention ont eu une croissance osseuse augmentée de façon très significative par rapport aux animaux témoins et au lot traité par le dichlorure de strontium. Dans ces deux lots, l'activité a été obtenue avec une moindre fixation du strontium dans l'os ( x 2 pour le lot avec le même dosage en strontium que le lot dichlorure de Sr, et x 1,5 pour le lot six fois moins dosé en strontium).

## Revendications

1. - Complexes organométalliques susceptibles d'être obtenus par réaction de:
- Au moins un cation de strontium,
- Le sitostérol ou un extrait végétal en contenant,
- Au moins un mono-, d'un di- ou d'un triglycéride répondant à la formule (I): 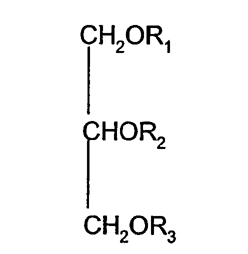
dans laquelle:
- R1 est un reste d'acide acyle gras en C14 à C24 saturé ou non, linéaire ou ramifié, de l'hydrogène, ou un mono-, un di- ou un tri- galactose ou glucose,
- R2 est un reste acyle d'acide gras en C2 à C18, linéaire ou ramifié, saturé ou non,
- R3 est un reste acyle d'acide gras en C14-C24 saturé ou non, linéaire ou ramifié, ou un atome d'hydrogène.

2. - Complexes selon la revendication 1, dans lesquels au moins un des groupements R1 ou R3 de la formule (I) ci-dessus est constitué d'un reste acyle d'acide oléique (C18: 1[cis]-9).

3. - Complexes selon la revendication 1 ou 2, dans lesquels R₂ présente une insaturation, de préférence il représente un reste d'acide oléique ou d'un de ses isomères de position de la double liaison (cis-6, 7, 9, 11, 12 et 13) ou d'un de ses isomères isoramifiés.

4. - Complexes selon la revendication 1 ou 2, dans lesquels R2 représente un groupe acétyle.

5. - Complexes selon l'une des revendications 1-4, dans lesquels R3 de la formule (I) représente l'atome d'hydrogène.

6. - Complexes selon l'une des revendications 1-5, dans lesquels le diglycéride est obtenu par isolement à partir de l'huile d'olive ou une huile riche en acide oléique, ou une fraction d'huile riche en acide oléique.

7. - Complexes selon l'une des revendications 1-6, dans lesquels le cation de strontium est choisi parmi les dihalogénures, plus spécifiquement le dichlorure, de strontium, les sulfates, les hydrates, des dérivés organiques de strontium comme les acétylacétonates, les alcoolates, en particulier le ranélate de strontium, et des complexes de strontium avec des solvants organiques.

8. - Composition pharmaceutique contenant au moins un complexe tel que défini dans l'une des revendications 1 à 7 et un véhicule, excipient ou support pharmaceutiquement acceptable.

9. - Utilisation de complexes tels que définis dans l'une des revendications 1-7 pour la préparation d'un médicament destiné à être utilisé comme agent régulateur ou stimulant de la croissance osseuse, en particulier dans le traitement ou la prévention des déficits ou des dysfonctionnements de la croissance osseuse, notamment dans le traitement ou la prévention de l'ostéoporose.

10. - Utilisation de complexes tels que définis dans l'une des revendications 1 à 7 pour la préparation d'un médicament destiné à être utilisé pour stimuler la fabrication de cellules souches hématopoïétiques, en particulier dans le traitement des pathologies sanguines s'accompagnant d'un déficit hématopoïétique, plus spécifiquement en complément des chimiothérapies anticancéreuses.

11. - Produits diététiques contenant au moins un complexe tel que défini dans l'une des revendications 1 à 7.

12. - Produits diététiques selon la revendication précédente pour la régularisation du métabolisme du calcium et/ou une prévention de la dégradation osseuse (densité et/ou qualité).

## Claims

1. - Organometallic complexes that can be obtained by reacting :
- at least one strontium cation,
- sitosterol or a plant extract containing same,
- at least one mono-, one di- or one triglyceride corresponding to formula (I): 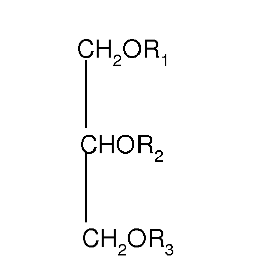
in which:
R1 is an acyl moiety of a C14 to C24 fatty acid, saturated or not, linear or branched, a hydrogen atom, or a mono-, di- or tri- galactose or glucose,
- R2 is an acyl moiety of a C2 to C18 fatty acid, linear or branched, saturated or not,
- R3 is an acyl moiety of a C14 to C24 fatty acid, saturated or not, linear or branched, or a hydrogen atom.

2. - Complexes according to claim 1, wherein at least one of the groups R1 or R3 in formula (I) hereinabove is composed of an acyl moiety of oleic acid (C18: 1[cis]-9).

3. - Complexes according to either one of claims 1 or 2, in which R₂ has an unsaturated bond, preferably it represents an oleic acid or one of the double bond positional isomers (cis-6, 7, 9, 11, 12 and 13) or one of the geometric isomers thereof.

4. - Complexes according to either one of claims 1 or 2, in which R2 represents an acetyl group.

5. - Complexes according to any one of claims 1 to 4, in which R3 in formula (I) represents the hydrogen atom.

6. - Complexes according to any one of claims 1 to 5, in which the diglyceride is obtained by isolation from olive oil or an oil rich in oleic acid, or an oil fraction rich in oleic acid.

7. - Complexes according to any one of claims 1 to 6, in which the strontium cation is selected in the group consisting of the dihalogenides, more specifically strontium dichloride, sulfates, hydrates, organic strontium derivatives such as acetylacetonates, alcoholates, in particular strontium ranelate, and complexes of strontium with organic solvents.

8. - Pharmaceutical composition containing at least one complex such as defined in any one of claims 1 to 7 and a pharmaceutically acceptable vehicle, excipient or support.

9. - Use of complexes such as defined in any one of claims 1 to 7 for preparing a medicament intended for use as a regulator or stimulant of bone growth, particularly in the treatment or prevention of deficiencies or dysfunctions of bone growth, specifically in the treatment or prevention of osteoporosis.

10. - Use of complexes such as defined in any one of claims 1 to 7 for preparing a medicament intended to stimulate the production of hematopoietic stem cells, particularly in the treatment of blood diseases involving a hematopoietic deficit, more specifically as an adjunct to anticancer chemotherapies.

11. - Dietetic products containing at least one complex such as defined in any one of claims 1 to 7.

12. - Dietetic products according to the previous claim for regulating calcium metabolism and/or preventing bone deterioration (density and/or quality).

## Patentansprüche

1. Organometallkomplexe, herstellbar durch eine Reaktion von:
- wenigstens einem Strontiumkation,
- Sitosterol oder einem Sitosterol-haltigen pflanzlichen Extrakt,
- wenigstens einem Mono-, Di- oder Triglycerid entsprechend der Formel (I): 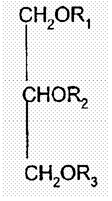
in der
- R1 ein gesättigter oder ungesättigter, linearer oder verzweigter C14 bis C24 Fettsäureacylrest, Wasserstoff oder eine Mono-, Di- oder Trigalactose oder -glucose ist,
- R2 ein gesättigter oder ungesättigter, linearer oder verzweigter C2 bis C18 Fettsäureacylrest ist,
- R3 ein gesättigter oder ungesättigter, linearer oder verzweigter C14 bis C24 Fettsäureacylrest oder ein Wasserstoffatom ist.

2. Komplexe gemäß Anspruch 1, in denen wenigstens eine der Gruppen R1 oder R3 der obigen Formel (I) aus einem Ölsäureacylrest (C18: 1[cis]-9) besteht.

3. Komplexe gemäß Anspruch 1 oder 2, in denen R₂ ungesättigt ist, vorzugsweise einen Ölsäurerest oder eines seiner Stellungsisomere der Doppelbindung (cis-6, 7, 9, 11, 12 und 13) oder eines seiner isoverzweigten Isomere darstellt.

4. Komplexe gemäß Anspruch 1 oder 2, in denen R2 eine Acetylgruppe darstellt.

5. Komplexe gemäß einem der Ansprüche 1 bis 4, in denen R3 der Formel (I) ein Wasserstoffatom darstellt.

6. Komplexe gemäß einem der Ansprüche 1 bis 5, in denen das Diglycerid durch Isolierung aus Olivenöl oder einem Ölsäure-reichen Öl oder einer Ölsäure-reichen Ölfraktion erhalten wird.

7. Komplexe gemäß einem der Ansprüche 1 bis 6, in denen das Strontiumkation aus Strontiumdihalogeniden, spezifischer dem Dichlorid, Strontiumsulfaten, Strontiumhydraten, organischen Strontiumderivaten, wie Acetylacetonaten, Alkoholaten, insbesondere Strontium-Ranelat, und Strontiumkomplexen mit organischen Lösemitteln ausgewählt ist.

8. Pharmazeutische Zusammensetzungen, die wenigstens einen wie in einem der Ansprüche 1 bis 7 definierten Komplex und ein Vehikulum, einen Exzipient oder einen pharmazeutisch verträglichen Träger enthalten.

9. Verwendung der wie in einem der Ansprüche 1 bis 7 definierten Komplexe für die Herstellung eines Medikaments, das als ein regulierendes oder stimulierendes Mittel für das Knochenwachstum verwendet werden soll, insbesondere bei der Behandlung oder Prävention von Defiziten oder Dysfunktionen des Knochenwachstums, besonders bei der Behandlung oder Prävention der Osteoporose.

10. Verwendung der wie in einem der Ansprüche 1 bis 7 definierten Komplexe für die Herstellung eines Medikaments, das zur Stimulierung der Produktion von hämatopoietischen Stammzellen verwendet werden soll, insbesondere bei der Behandlung von Blutkrankheiten, die mit einem hämatopoietischen Defizit einhergehen, spezifischer als Ergänzung von Chemotherapien zur Krebsbekämpfung.

11. Diätetische Produkte, die wenigstens einen wie in einem der Ansprüche 1 bis 7 definierten Komplex enthalten.

12. Diätetische Produkte gemäß dem vorhergehenden Anspruch für die Regulierung des Calcium-Stoffwechsels und/oder für eine Prävention des Knochenabbaus (Dichte und/oder Qualität).
